# EUROPEAN PATENT SPECIFICATION

(11) **EP 3 393 432 B1**
(45) Date of publication and mention of the grant of the patent: **15.04.2020**
(21) Application number: 16822431.9
(22) Date of filing: 16.12.2016
(51) Int. Cl.: A61K 8/31, A61K 8/41, A61Q 1/02, A61Q 1/06, A61Q 1/10, A61K 8/81, A61K 8/898

(54) **COSMETIC PROCESS FOR TREATING KERATIN MATERIALS WITH AN ACRYLIC POLYMER BEARING MALEIC ANHYDRIDE GROUPS**
KOSMETISCHES VERFAHREN ZUR BEHANDLUNG VON KERATINMATERIALIEN MIT EINEM ACRYLPOLYMER MIT MALEINSÄUREANHYDRIDGRUPPEN
PROCÉDÉ COSMÉTIQUE POUR LE TRAITEMENT DES MATIÈRES KÉRATINIQUES À L'AIDE D'UN POLYMÈRE ACRYLIQUE PORTANT DES GROUPES ANHYDRIDE MALÉIQUE

(30) Priority: 22.12.2015 FR 1563111
(43) Date of publication of application: 31.10.2018
(73) Proprietor: L'Oréal, 75008 Paris (FR)
(72) Inventor: LION, Bertrand, 93601 Aulnay-sous-Bois (FR); PORTAL, Julien, 93601 Aulnay-sous-Bois (FR)
(74) Representative: L'Oreal
(86) International application number: PCT/EP2016/081347
(87) International publication number: WO 2017/108596

(56) References cited:
- FR-A1- 2 972 630
- FR-A1- 3 014 875
- US-A- 5 185 143
- US-A1- 2010 120 931
- US-A1- 2014 227 210
- US-A1- 2015 044 790

## Description

The present invention relates to a cosmetic process for treating keratin materials using a maleic anhydride acrylic polymer and a polyamine compound, and also to a kit for performing said process.

Cosmetic products often require the use of a film-forming polymer to obtain a deposit of the product on keratin materials that has good cosmetic properties. In particular, it is necessary for the film-forming deposit to have good persistence, in particular for the deposit not to transfer during contact with the fingers, clothing, a glass or a cup, and also good persistence on contact with water, especially rain or during showering or alternatively perspiration. Skin sebum may also damage the film-forming deposit.

It is known to those skilled in the art to use polymers in order to obtain these good persistence properties throughout the day. These polymers are of very different chemical nature and are generally conveyed either in a fatty phase or in an aqueous phase. Examples that may be mentioned include silicone resins, polyacrylates and latices.

Document US5185143 discloses a polymer obtained by polymerisation of maleic anhydride with vinyl acetate and isobornyl (meth)acrylate. It comprises less than 50 % by weight of isobornyl(meth)acrylate. Said polymer is used as a hair fixative.

Although these polymers do indeed afford persistence properties, in particular transfer resistance, they may have a certain level of discomfort: for example, after applying the product, they may have a tacky aspect.

There is thus still a need for polymers that can afford good persistence properties while at the same time maintaining a certain level of comfort during use.

The inventors have discovered that a particular maleic anhydride acrylic polymer combined with a particular polyamine compound makes it possible to obtain a deposit on keratin materials that has good film-forming properties.
The film-forming deposit obtained has good water resistance and also good resistance to oil (especially to olive oil) and to sebum.
This particular acrylic polymer is readily conveyable in a hydrocarbon-based oil such as isododecane.
Furthermore, the film-forming deposit has good tack-resistance and transfer-resistance properties, especially when the film is touched with the fingers: the deposit obtained thus has good persistence properties.

Furthermore, when the maleic anhydride acrylic polymer is formulated with a non-volatile oil (often used in makeup products), for instance 2-octylethanol, the process according to the invention makes it possible to obtain a film-forming deposit which has good persistence, transfer-resistance, tack-resistance, water-resistance, oil-resistance and sebum-resistance properties.
This maleic anhydride acrylic polymer combined with said polyamine compound forms a film-forming deposit that is suitable for making up the skin or the lips or the eyelashes, such as foundations, lipsticks or mascaras, or for fixing the hair.

Document US2015/0044790 discloses said such a polymer. It is used with diethylaminopropylamine as a photosensitive resin in photolithography.

More precisely, a subject of the present invention is a treatment process, especially a cosmetic treatment process, in particular for caring for or making up keratin materials, comprising the sequential application to keratin materials of a composition, especially a cosmetic composition, comprising a maleic anhydride acrylic polymer and of a polyamine compound, or a cosmetic composition containing same,
said maleic anhydride acrylic polymer being able to be obtained by polymerization of:
(a) 50% to 90% by weight, relative to the total weight of monomers, of isobornyl (meth)acrylate
(b) 1% to 50% by weight of maleic anhydride
(c) 0 to 49% by weight of additional (meth)acrylate monomer chosen from:
   (i) linear or branched, saturated or unsaturated C₁-C₂₀ alkyl (meth)acrylates, optionally interrupted with one or more non-adjacent heteroatoms chosen from O and S or with a group NR, R being a C₁-C₄ alkyl group, optionally substituted with a phenyl or furfuryl group;
   (ii) saturated C₄-C₈ cycloalkyl (meth)acrylates optionally interrupted with O or NH;
   said amine compound being chosen from polyamine compounds bearing several primary amine and/or secondary amine groups, the amine compound not being an alkoxysilane.

The process according to the invention is suitable for caring for or making up keratin materials, such as the skin, the lips, the eyelashes, the hair or the nails.
The process according to the invention is also suitable for shaping the hair, especially for styling. The hair fixing shows good water resistance.
A subject of the invention is also a kit comprising a first composition comprising said maleic anhydride acrylic polymer as described previously and comprising a physiologically acceptable medium, and a second composition comprising an amine compound as described previously and comprising a physiologically acceptable medium, the first and second compositions each being packaged in a separate packaging assembly.
The composition packaging assembly is, in a known manner, any packaging that is suitable for storing cosmetic compositions (in particular a bottle, tube, spray bottle or aerosol bottle).
Such a kit allows the skin treatment process according to the invention to be performed.

The maleic anhydride acrylic polymer used according to the invention comprises (or is derived from the polymerization of) an isobornyl (meth)acrylate, maleic anhydride and optionally an additional acrylate monomer as defined previously. Advantageously, the maleic anhydride acrylic polymer is formed essentially from these monomers in the contents described hereinabove or hereinbelow.

Advantageously, the polymer used according to the invention is derived from the polymerization of:
(a) 50% to 90% by weight, relative to the total weight of monomers, of isobornyl (meth)acrylate
(b) 5% to 50% by weight of maleic anhydride
(c) 0 to 30% by weight of additional (meth)acrylate monomer as described previously.

Advantageously, the polymer used according to the invention is derived from the polymerization of:
(a) 50% to 80% by weight, relative to the total weight of monomers, of isobornyl (meth)acrylate
(b) 5% to 30% by weight of maleic anhydride
(c) 15 to 30% by weight of additional (meth)acrylate monomer as described previously.

Advantageously, the polymer used according to the invention is derived from the polymerization of:
(a) 50% to 80% by weight, relative to the total weight of monomers, of isobornyl (meth)acrylate
(b) 5% to 25% by weight of maleic anhydride
(c) 15 to 30% by weight of additional (meth)acrylate monomer as described previously.

Advantageously, the polymer used according to the invention is derived from the polymerization of:
(a) 50% to 80% by weight, relative to the total weight of monomers, of isobornyl (meth)acrylate
(b) 5% to 15% by weight of maleic anhydride
(c) 15 to 30% by weight of additional (meth)acrylate monomer as described previously.

Advantageously, the polymer used according to the invention is derived from the polymerization of:
(a) 60% to 80% by weight, relative to the total weight of monomers, of isobornyl (meth)acrylate
(b) 5% to 12% by weight of maleic anhydride
(c) 15 to 30% by weight of additional (meth)acrylate monomer as described previously.

Advantageously, the polymer used according to the invention is derived from the polymerization of:
(a) 60% to 80% by weight, relative to the total weight of monomers, of isobornyl (meth)acrylate
(b) 5% to 12% by weight of maleic anhydride
(c) 18 to 30% by weight of additional (meth)acrylate monomer as described previously.

The additional (meth)acrylate monomer is preferably chosen from C₆-C₁₆ alkyl (meth)acrylates, and preferentially chosen from C₆-C₁₆ alkyl acrylates.
As examples of C₆-C₁₆ alkyl (meth)acrylates, mention may be made of hexyl (meth)acrylate, octyl (meth)acrylate, 2-ethylhexyl (meth)acrylate, decyl (meth)acrylate and lauryl (meth)acrylate. 2-Ethylhexyl acrylate is preferably used.

Preferably, the polymer used according to the invention comprises, or consists of, isobornyl acrylate, 2-ethylhexyl acrylate and maleic anhydride.

A particularly preferred polymer used according to the invention is derived from the polymerization of:
(a) 50% to 80% by weight, relative to the total weight of monomers, of isobornyl (meth)acrylate
(b) 5% to 30% by weight of maleic anhydride
(c) 15% to 30% by weight of C₆-C₁₆ alkyl acrylate monomer.

A particularly preferred polymer used according to the invention is derived from the polymerization of:
(a) 50% to 80% by weight, relative to the total weight of monomers, of isobornyl (meth)acrylate
(b) 5% to 25% by weight of maleic anhydride
(c) 15% to 30% by weight of C₆-C₁₆ alkyl acrylate monomer.

A particularly preferred polymer used according to the invention is derived from the polymerization of:
(a) 50% to 80% by weight, relative to the total weight of monomers, of isobornyl (meth)acrylate
(b) 5% to 15% by weight of maleic anhydride
(c) 15% to 30% by weight of C₆-C₁₆ alkyl acrylate monomer.

A particularly preferred polymer used according to the invention is derived from the polymerization of:
(a) 60% to 80% by weight, relative to the total weight of monomers, of isobornyl (meth)acrylate
(b) 5% to 12% by weight of maleic anhydride
(c) 15% to 30% by weight of C₆-C₁₆ alkyl acrylate monomer.

A particularly preferred polymer used according to the invention is derived from the polymerization of:
(a) 60% to 80% by weight, relative to the total weight of monomers, of isobornyl (meth)acrylate
(b) 5% to 12% by weight of maleic anhydride
(c) 18% to 30% by weight of C₆-C₁₆ alkyl acrylate monomer.

Advantageously, the polymer used according to the invention consists of the monomers described previously.

Advantageously, the polymer used according to the invention is nonionic.

Preferably, the maleic anhydride acrylic polymer used according to the invention has a weight-average molecular weight ranging from 5000 to 1 000 000 g/mol, preferably ranging from 10 000 to 500 000 g/mol and preferentially ranging from 15 000 to 350 000 g/mol.
The molecular weight may especially be determined by steric exclusion chromatography, with THF eluent, polystyrene standard, 2414 refractometric detector from Waters.

The copolymer may be a random, alternating (block) or gradient polymer. Preferably, the copolymer is random.

The copolymer used according to the invention may be prepared by radical polymerization of the monomers described previously, especially as a mixture or added sequentially during the polymerization, especially using an organic solvent with a boiling point of greater than or equal to 60°C, for instance isododecane, ethanol, ethyl acetate, tetrahydrofuran, methyltetrahydrofuran or methyl ethyl ketone. The organic solvent makes it possible to dissolve the monomers used and the polymer formed.
The polymerization is especially performed in the presence of a radical initiator especially of peroxide type (for example tert-butyl peroxy-2-ethylhexanoate: Trigonox 21S; 2,5-dimethyl-2,5-bis(2-ethylhexanoylperoxy)hexane:Trigonox 141; tert-butyl peroxypivalate: Trigonox 25C75 from AkzoNobel) or of azo type, for example (AIBN: azobisisobutyronitrile; V50: 2,2'-azobis(2-amidinopropane) dihydrochloride).

The polymerization may be performed at a temperature ranging from 60 to 100°C, and preferably ranging from 60 to 85°C.
The polymerization time may be about 24 hours.

The polymer used according to the invention may be used in a composition comprising a physiologically acceptable medium, in particular in a cosmetic composition.

The term "physiologically acceptable medium" means a medium that is compatible with human keratin materials.

The term "cosmetic composition" is understood to mean a composition that is compatible with keratin materials, which has a pleasant colour, odour and feel and which does not cause unacceptable discomfort (stinging, tautness or redness) liable to discourage the consumer from using it.

The maleic anhydride acrylic polymer as defined previously may be present in the composition according to the invention in a content ranging from 0.1% to 40% by weight, relative to the total weight of the composition, preferably from 0.5% to 35% by weight of active material, preferentially ranging from 1% to 30% by weight, especially ranging from 5% to 30% by weight and more preferentially ranging from 10% to 30% by weight.

The polyamine compound used in the process according to the invention is chosen from polyamine compounds bearing several primary amine and/or secondary amine groups, the polyamine compound not being an alkoxysilane.

The term "alkoxysilane compound" means a compound comprising only one Si atom, this atom being bonded to at least one (especially from 1 to 3) group -OR, R being a linear or branched alkyl group, comprising from 1 to 6 carbon atoms.

According to a first embodiment of the invention, the polyamine compound is a compound comprising from 2 to 20 carbon atoms, in particular a non-polymeric compound. The term "non-polymeric compound" means a compound which is not directly obtained via a monomer polymerization reaction The polyamine compound is preferably a diamine compound.

Polyamine compounds that may be mentioned include N-methyl-1,3-diaminopropane, N-propyl-1,3-diaminopropane, N-isopropyl-1,3-diaminopropane, N-cyclohexyl-1,3-diaminopropane, 2-(3-aminopropylamino)ethanol, 3-(2-aminoethyl)aminopropylamine, bis(3-aminopropyl)amine, methylbis(3-aminopropyl)amine, N-(3-aminopropyl)-1,4-diaminobutane, N,N-dimethyldipropylenetriamine, 1,2-bis(3-aminopropylamino)ethane, N,N'-bis(3-aminopropyl)-1,3-propanediamine, ethylenediamine, 1,3-propylenedimaine, 1,4-butylenediamine, lysine, cystamine, xylenediamine, tris(2-aminoethyl)amine and spermidine. Preferably, the amine compound is chosen from ethylenediamine, 1,3-propylenediamine and 1,4-butylenediamine. Preferentially, the polyamine compound is ethylenediamine.

According to a second embodiment, the polyamine compound may be chosen from amine-based polymers.

The amine-based polymer may have a weight-average molecular weight ranging from 500 to 1 000 000, preferably ranging from 500 to 500 000, and preferentially ranging from 500 to 100 000.

As amine-based polymer, use may be made of poly((C₂-C₅)alkyleneimines), and in particular polyethyleneimines and polypropyleneimines, especially poly(ethyleneimine)s (for example the product sold under the reference 46,852-3 by the company Aldrich Chemical); poly(allylamine) (for example the product sold under the reference 47,913-6 by the company Aldrich Chemical); polyvinylamines and copolymers thereof, in particular with vinylamides; mention may in particular be made of vinylamine/vinylformamide copolymers such as those sold under the name Lupamin® 9030 by the company BASF; polyamino acids bearing NH₂ groups, such as polylysine, for example the product sold by the company JNC Corporation (formerly Chisso); aminodextran, such as the product sold by the company CarboMer Inc; amino polyvinyl alcohol, such as the product sold by the company CarboMer Inc, acrylamidopropylamine-based copolymers; chitosans;
polydimethylsiloxanes comprising primary amine groups at the chain end or on side chains, for example aminopropyl side or end groups, for instance those of formula (A) or (B) or (C): 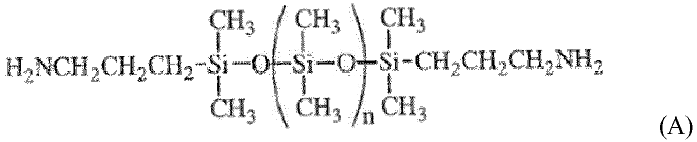 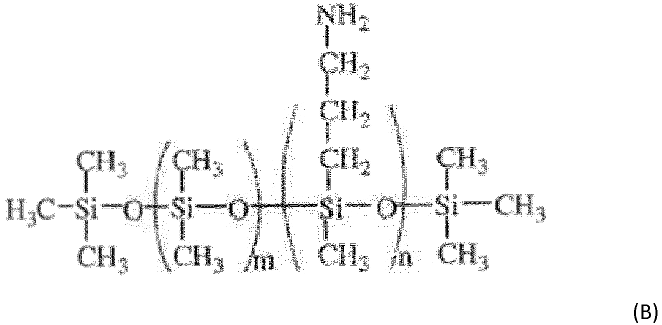 H₂NCH₂CH₂CH₂-Si(CH₃)₂-O-[Si(CH₃)₂-O]ₙ-Si(CH₃)₂C₄H₉ (C) in formula (A): the value of n is such that the weight-average molecular weight of the silicone is between 500 and 55 000. As an example of aminosilicone (A), mention may be made of those sold under the names DMS-A11, DMS-A12, DMS-A15, DMS-A21, DMS-A31, DMS-A32 and DMS-A35 by the company Gelest; reference 481688 from Aldrich.
in formula (B), the values of n and m are such that the weight-average molecular weight of the silicone is between 1000 and 55 000. As examples of silicone (B), mention may be made of those sold under the names AMS-132, AMS-152, AMS-162, AMS-163, AMS-191 and AMS-1203 by the company Gelest.
in formula (C), the value of n is such that the weight-average molecular weight of the silicone is between 500 and 3000. As an example of silicone (C), mention may be made of those sold under the names MCR-A11 and MCR-A12 by the company Gelest;
amodimethicones of formula (D): 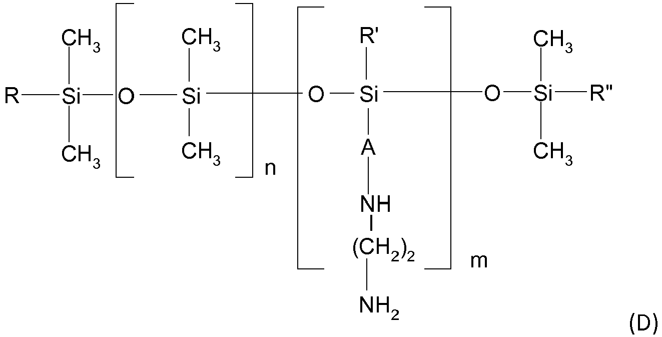 in which R, R' and R", which may be identical or different, each represent a C₁-C₄ alkyl or hydroxyl group, A represents a C₃ alkylene group and m and n are such that the weight-average molecular mass of the compound is between 5000 and 500 000 approximately;
amodimethicones of formula (K): 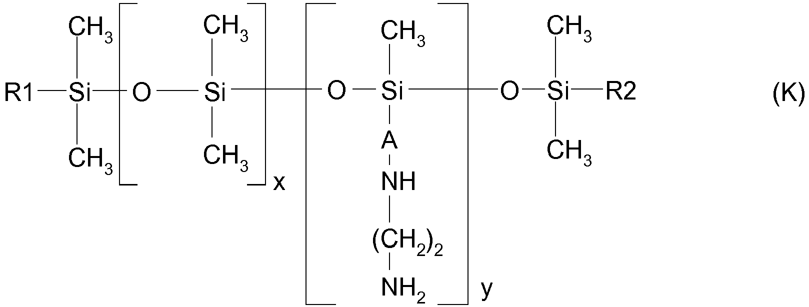 in which:
- R1 and R2, which may be identical or different, preferably identical, represent a linear or branched, saturated or unsaturated alkyl group comprising from 6 to 30 carbon atoms, preferably from 8 to 24 carbon atoms and preferentially from 12 to 20 carbon atoms,
- A represents a linear or branched alkylene radical group containing from 2 to 8 carbon atoms,
- x and y are integers ranging from 1 to 5000; preferably, x ranges from 10 to 2000 and especially from 100 to 1000; preferably, y ranges from 1 to 100.

Preferably, A comprises from 3 to 6 carbon atoms, in particular 4 carbon atoms; preferably, A is branched. A may be a divalent radical chosen from: -CH₂CH₂CH₂-and -CH₂CH(CH₃)CH₂-.

Preferably, R1 and R2, which may be identical or different, represent a saturated linear alkyl group comprising from 6 to 30 carbon atoms, preferentially from 8 to 24 carbon atoms and especially from 12 to 20 carbon atoms, for instance a dodecyl, tetradecyl, pentadecyl, hexadecyl, heptadecyl, octadecyl, nonadecyl or eicosyl group. Advantageously, R1 and R2 represent a mixture of hexadecyl (cetyl) and octadecyl (stearyl) radicals (mixture also known as cetearyl).

Preferentially, for the amodimethicone of formula (K):
- x ranges from 10 to 2000 and especially from 100 to 1000;
- y ranges from 1 to 100;
- A comprises from 3 to 6 carbon atoms, and in particular 4 carbon atoms; preferably, A is branched; preferentially, A is chosen from the divalent radicals: -CH₂CH₂CH₂- and
- CH₂CH(CH₃)CH₂-; and
- R1 and R2, which may be identical or different, represent a saturated linear radical comprising from 6 to 30 carbon atoms, preferably from 8 to 24 carbon atoms and especially from 12 to 20 carbon atoms, for instance a dodecyl, tetradecyl, pentadecyl, hexadecyl, heptadecyl, octadecyl, nonadecyl or eicosyl group. Advantageously, R1 and R2 represent a mixture of hexadecyl (cetyl) and octadecyl (stearyl) radicals (mixture also known as cetearyl).

As amodimethicone of formula (K), use may be made of bis-cetearyl amodimethicone (INCI name), especially the product sold under the name Silsoft® AX by the company Momentive Performance Materials.

The polyether amines known especially under the reference Jeffamine® from the company Huntsman; and especially:
polyethylene glycol and/or polypropylene glycol α,ω-diamines (bearing an amine function at the end of the chain), which may comprise from 2 to 80 units derived from propylene oxide, or which may comprise from 2 to 50 units derived from ethylene oxide and from 1 to 10 units derived from propylene oxide, for instance the products sold under the names Jeffamine® D-230, D-400, D-2000, D-4000, ED-600, ED-9000, ED-2003;
polytetrahydrofuran (or polytetramethylene glycol) α,ω-diamines;
polybutadiene α,ω-diamines;
polyamidoamine (PANAM) dendrimers bearing amine end functions;
poly(meth)acrylates or poly(meth)acrylamides bearing primary or secondary amine side functions, such as poly(3-aminopropyl)methacrylamide or poly(2-aminoethyl) methacrylate.

As amine-based polymer, use is preferably made of polydimethylsiloxanes comprising primary amine groups at the chain end or on side chains.
Preferentially, polydimethylsiloxanes comprising aminopropyl end groups at the chain end are used.

Advantageously, the polyamine compounds used in the process according to the invention are chosen from ethylenediamine, polydimethylsiloxanes comprising primary amine groups at the chain end or on side chains, amodimethicones of formula (K), in particular bis-cetearyl amodimethicone; polyethylene glycol and/or polypropylene glycol α,ω-diamines;ethylenediamine, 1,3-propylenediamine, 1,4-butylenediamine, preferably ethylenediamine.

Preferentially, the polyamine compounds used in the process according to the invention are chosen from ethylenediamine, polydimethylsiloxanes comprising aminopropyl end groups at the chain end, bis-cetearyl amodimethicone, polyethylene glycol/polypropylene glycol α,ω-diamine copolymers comprising from 2 to 50 units derived from ethylene oxide and from 1 to 10 units derived from propylene oxide.

Advantageously, the polyamine compound is used in a mole ratio of amine group of the polyamine compound/maleic anhydride group of the acrylic polymer ranging from 0.01 to 10, preferably ranging from 0.1 to 5, preferentially ranging from 0.1 to 2 and more preferentially ranging from 0.1 to 1.

On contact with the acrylic polymer, the polyamine compound reacts with the maleic anhydride functions to form a crosslinked polymer, for example in the following manner:

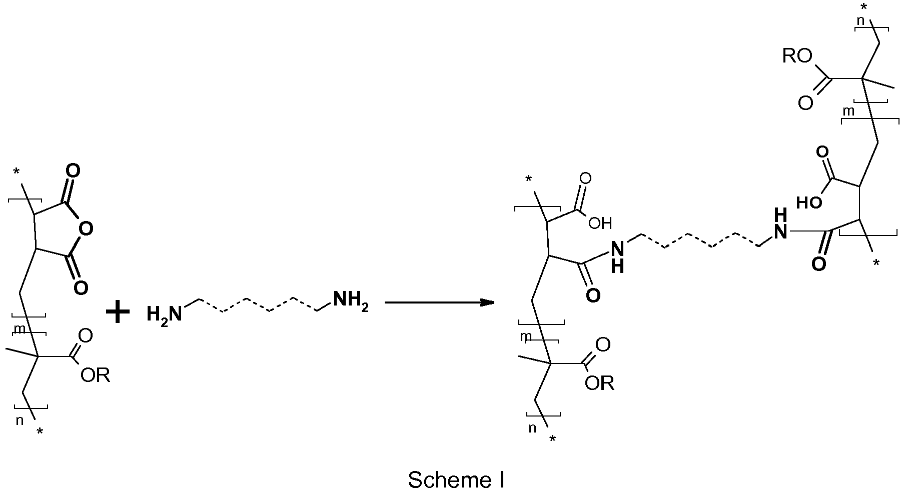

Such a crosslinked polymer is novel and thus also forms the subject of the present invention.

The crosslinked polymer may thus be obtained by reacting said amine compound with the maleic anhydride acrylic polymer described previously. Some or all of the anhydride groups react with the NH or NH₂ group of the polyamine compound and form a unit bearing an amide group and a carboxylic acid group as described in scheme I.

The composition(s) used according to the invention are generally suitable for topical application to keratin materials, and thus generally comprise a physiologically acceptable medium, i.e. a medium that is compatible with the skin and/or its integuments. It is preferably a cosmetically acceptable medium, i.e. a medium which has a pleasant colour, odour and feel and which does not cause any unacceptable discomfort (stinging, tautness or redness) liable to discourage the consumer from using this composition.

Advantageously, the process according to the invention is performed under ambient conditions, in particular at an ambient temperature that may range from 15°C to 30°C, preferably ranging from 18°C to 25°C.

According to a preferred embodiment of the invention, the composition comprising the maleic anhydride acrylic polymer may contain a hydrocarbon-based oil.
The hydrocarbon-based oil is an oil that is liquid at room temperature (25°C).
The term "hydrocarbon-based oil" means an oil formed essentially from, or even constituted of, carbon and hydrogen atoms, and optionally oxygen and nitrogen atoms, and not containing any silicon or fluorine atoms. It may contain alcohol, ester, ether, carboxylic acid, amine and/or amide groups.

The hydrocarbon-based oil may be volatile or non-volatile.

The hydrocarbon-based oil may be chosen from:
hydrocarbon-based oils containing from 8 to 14 carbon atoms, and especially:
- branched C₈-C₁₄ alkanes, for instance C₈-C₁₄ isoalkanes of petroleum origin (also known as isoparaffins), for instance isododecane (also known as 2,2,4,4,6-pentamethylheptane), isodecane and, for example, the oils sold under the trade name Isopar or Permethyl,
- linear alkanes, for instance n-dodecane (C12) and n-tetradecane (C14) sold by Sasol under the respective references Parafol 12-97 and Parafol 14-97, and also mixtures thereof, the undecane-tridecane mixture, the mixtures of n-undecane (C11) and of n-tridecane (C13) obtained in Examples 1 and 2 of patent application WO 2008/155 059 from the company Cognis, and mixtures thereof,
- short-chain esters (containing from 3 to 8 carbon atoms in total) such as ethyl acetate, methyl acetate, propyl acetate or n-butyl acetate,
- hydrocarbon-based oils of plant origin such as triglycerides consisting of fatty acid esters of glycerol, the fatty acids of which may have chain lengths varying from C₄ to C₂₄, these chains possibly being linear or branched, and saturated or unsaturated; these oils are especially heptanoic or octanoic acid triglycerides, or alternatively wheatgerm oil, sunflower oil, grapeseed oil, sesame seed oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passion-flower oil and musk rose oil; shea butter; or else caprylic/capric acid triglycerides, for instance those sold by the company Stéarineries Dubois or those sold under the names Miglyol 810®, 812® and 818® by the company Dynamit Nobel,
- synthetic ethers having from 10 to 40 carbon atoms;
- linear or branched hydrocarbons of mineral or synthetic origin, such as petroleum jelly, polydecenes, hydrogenated polyisobutene such as Parleam®, squalane and liquid paraffins, and mixtures thereof,
- synthetic esters such as oils of formula R₁COOR₂ in which R₁ represents a linear or branched fatty acid residue containing from 1 to 40 carbon atoms and R₂ represents an, in particular, branched hydrocarbon-based chain containing from 1 to 40 carbon atoms, on the condition that R₁ + R₂ ≥ 10, for instance purcellin oil (cetostearyl octanoate), isopropyl myristate, isopropyl palmitate, C₁₂ to C₁₅ alkyl benzoates, hexyl laurate, diisopropyl adipate, isononyl isononanoate, 2-ethylhexyl palmitate, isostearyl isostearate, 2-hexyldecyl laurate, 2-octyldecyl palmitate, 2-octyldodecyl myristate, alkyl or polyalkyl heptanoates, octanoates, decanoates or ricinoleates such as propylene glycol dioctanoate; hydroxylated esters such as isostearyl lactate, diisostearyl malate and 2-octyldodecyl lactate; polyol esters and pentaerythritol esters,
- fatty alcohols that are liquid at room temperature, with a branched and/or unsaturated carbon-based chain containing from 12 to 26 carbon atoms, for instance octyldodecanol, isostearyl alcohol, oleyl alcohol, 2-hexyldecanol, 2-butyloctanol and 2-undecylpentadecanol.

Advantageously, the hydrocarbon-based oil is apolar (thus formed solely from carbon and hydrogen atoms).

The hydrocarbon-based oil is preferably chosen from hydrocarbon-based oils containing from 8 to 14 carbon atoms, in particular the apolar oils described previously.

Preferentially, the hydrocarbon-based oil is isododecane.
The composition comprising the polymer may contain, in addition to the hydrocarbon-based oil, a silicone oil. The term "silicone oil" means an oil comprising at least one silicon atom and especially at least one Si-O group. The silicone oil may be volatile or non-volatile.
The term "volatile oil" means an oil (or non-aqueous medium) that is capable of evaporating on contact with the skin in less than one hour, at room temperature and at atmospheric pressure. The volatile oil is a volatile cosmetic oil, which is liquid at room temperature, especially having a non-zero vapour pressure, at room temperature and atmospheric pressure, in particular having a vapour pressure ranging from 0.13 Pa to 40 000 Pa (10⁻³ to 300 mmHg), preferably ranging from 1.3 Pa to 13 000 Pa (0.01 to 100 mmHg) and preferentially ranging from 1.3 Pa to 1,300 Pa (0.01 to 10 mmHg).
The term "non-volatile oil" means an oil with a vapour pressure of less than 0.13 Pa.

Volatile silicone oils that may be mentioned include volatile linear or cyclic silicone oils, especially those with a viscosity ≤ 8 centistokes (cSt) (8 x 10⁻⁶ m²/s), and especially having from 2 to 10 silicon atoms and in particular from 2 to 7 silicon atoms, these silicones optionally comprising alkyl or alkoxy groups having from 1 to 10 carbon atoms. As volatile silicone oil that may be used in the invention, mention may be made especially of dimethicones with viscosities of 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

As non-volatile silicone oils, mention may be made of linear or cyclic non-volatile polydimethylsiloxanes (PDMSs); polydimethylsiloxanes comprising alkyl, alkoxy or phenyl groups, which are pendant or at the end of a silicone chain, these groups containing from 2 to 24 carbon atoms; phenyl silicones, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates.

Advantageously, the composition may comprise a hydrocarbon-based oil in a content ranging from 60% to 100% by weight relative to the total weight of the oils present in the composition and from 0 to 40% by weight of silicone oil. According to a preferred embodiment of the invention, the composition contains as oil only a hydrocarbon-based oil.

The composition according to the invention may comprise a cosmetic additive chosen from fragrances, preserving agents, fillers, UV-screening agents, oils, waxes, surfactants, moisturizers, vitamins, ceramides, antioxidants, free-radical scavengers, polymers, thickeners and dyestuffs.

The composition according to the invention may also comprise a dyestuff such as pulverulent dyestuffs, liposoluble dyes or water-soluble dyes. This dyestuff may be present in a content ranging from 0.01% to 30% by weight, relative to the total weight of the composition.

The pulverulent dyestuffs may be chosen from pigments and nacres.

The pigments may be white or coloured, mineral and/or organic, and coated or uncoated. Among the mineral pigments that may be mentioned are titanium dioxide, optionally surface-treated, zirconium, zinc or cerium oxide, and also iron or chromium oxide, manganese violet, ultramarine blue, chromium hydrate and ferric blue. Among the organic pigments that may be mentioned are carbon black, pigments of D&C type and lakes based on cochineal carmine or on barium, strontium, calcium or aluminium.

The nacres may be chosen from white nacreous pigments such as mica coated with titanium or with bismuth oxychloride, coloured nacreous pigments such as titanium mica with iron oxides, titanium mica with in particular ferric blue or chromium oxide, titanium mica with an organic pigment of the abovementioned type, and also nacreous pigments based on bismuth oxychloride.

The liposoluble dyes are, for example, Sudan Red, D&C Red 17, D&C Green 6, β-carotene, soybean oil, Sudan Brown, D&C Yellow 11, D&C Violet 2, D&C Orange 5, quinoline yellow and annatto. The water-soluble dyes are, for example, beetroot juice or methylene blue.

Advantageously, the composition according to the invention is a skincare composition.

The composition according to the invention may be a makeup composition such as a foundation, a lipstick or a liner.

According to one embodiment, the composition according to the invention is a makeup composition and comprises a volatile oil and a non-volatile oil as described previously. In particular, the makeup composition may comprise a hydrocarbon-based volatile oil and a hydrocarbon-based non-volatile oil.

According to one embodiment, the composition according to the invention is a hair fixing composition.
According to one embodiment, the composition used in the process according to the invention is an anhydrous composition. The term "anhydrous composition" means a composition containing less than 2% by weight of water, or even less than 0.5% of water, and is especially free of water. Where appropriate, such small amounts of water may especially be introduced by ingredients of the composition that may contain residual amounts thereof.

According to a first embodiment of the process according to the invention, a composition, especially a cosmetic composition, comprising the acrylic polymer is first applied to the keratin materials, and said polyamine compound or a cosmetic composition containing same is then applied. The application of the polyamine compound may be performed after a time of between 5 minutes and one hour after having applied the acrylic polymer to the keratin materials.
According to a second embodiment of the process according to the invention, said polyamine compound, or a cosmetic composition containing same, is first applied to the keratin materials, and the composition, especially the cosmetic composition, comprising the acrylic polymer is then applied. The application of the acrylic polymer may be performed after a time of between 5 minutes and one hour after having applied said polyamine compound to the keratin materials.

The invention will now be described with reference to the examples that follow.

### Example 1: Isobornyl acrylate/2-ethylhexyl acrylate/maleic anhydride copolymer (70/20/10 by weight) - Polymer 1

70 g of isobornyl acrylate, 20 g of 2-ethylhexyl acrylate and 10 g of maleic anhydride were placed in a jacketed 1-litre reactor equipped with a stirring anchor. A mixture of 70 g of isododecane and 30 g of ethyl acetate was then added. The medium was brought to a temperature of 40°C with stirring (150 rpm) and was sparged with argon for 10 minutes, followed by addition of 0.5 g of initiator tert-butyl peroxy-2-ethylhexanoateTrigonox 21S (Trigonox® 21S from Akzo Nobel).

The heating of the jacket was set at 90°C for 7 hours at 150 rpm.
The medium was then diluted with 300 g of isododecane, and then concentrated by distillation to remove the ethyl acetate and the unreacted maleic anhydride.
A solution containing 30% by weight of the copolymer in isododecane (yield of greater than 90%) was finally obtained.
The polymer obtained has a molecular weight (Mw) of close to 200 000 g/mol.

### Example 2: Isobornyl acrylate/2-ethylhexyl acrylate/maleic anhydride copolymer (65/25/10 by weight) - Polymer 2

The polymer was prepared according to the procedure of Example 1, using 65 g of isobornyl acrylate, 25 g of 2-ethylhexyl acrylate and 10 g of maleic anhydride. A solution containing 30% by weight of the copolymer in isododecane (yield of greater than 90%) was finally obtained.
The polymer obtained has a molecular weight (Mw) of close to 200 000 g/mol.

### Example 3: Isobornyl acrylate/2-ethylhexyl acrylate/maleic anhydride copolymer (75/20/5 by weight) - Polymer 3

The polymer was prepared according to the procedure of Example 1, using 75 g of isobornyl acrylate, 20 g of 2-ethylhexyl acrylate and 5 g of maleic anhydride. A solution containing 30% by weight of the copolymer in isododecane (yield of greater than 90%) was finally obtained.
The polymer obtained has a molecular weight (Mw) of close to 200 000 g/mol.

### Example 4: Isobornyl acrylate/2-ethylhexyl acrylate/maleic anhydride copolymer (60/20/20 by weight) - Polymer 4

The polymer was prepared according to the procedure of Example 1, using
60 g of isobornyl acrylate, 20 g of 2-ethylhexyl acrylate and 20 g of maleic anhydride.
A solution containing 36% by weight of the copolymer in isododecane (yield of greater than 90%) was finally obtained.
The polymer obtained has a molecular weight (Mw) of close to 200 000 g/mol.

### Comparative Examples 5 to 10: Cosmetic evaluation of makeup compositions

6 makeup compositions (lipstick, foundation) of base coat containing the polymer of Example 1 and of top coat described below (Examples 6, 8, 10 according to the invention: top coat with 3-aminopropyl-terminated polydimethylsiloxane; Examples 5, 7, 9, 13 outside the invention: top coat without 3-aminopropyl-terminated polydimethylsiloxane) were prepared.

Each base coat composition was applied onto a skin equivalent support made of elastomer by producing a deposit with a wet thickness of 100 µm, which was left to dry at room temperature (25°C) for 24 hours.

The top coat composition was then applied onto each dry base coat deposit by producing a deposit with a wet thickness of 100 µm, which was left to dry at room temperature (25°C) for 24 hours.

The state of the film obtained was then observed.

The resistance of the film obtained was evaluated by separately applying 0.5 ml of water, 0.5 ml of olive oil and 0.5 ml of sebum; after 5 minutes of contact, the surface of the film was rubbed with cotton wool and the state of the film was then observed.

The tackiness of the film and its capacity for transferring or not transferring on touching the film with a finger were also evaluated.

The evaluation was made in the following manner:
+++: very efficient evaluated cosmetic property
++: moderately efficient evaluated cosmetic property
+: sparingly efficient evaluated cosmetic property
0: inefficient evaluated cosmetic property

The following results were obtained:

| | Example 5 | Example 6 (invention) | Example 7 | Example 8 (invention) | Example 9 | Example 10 (invention) |
|---|---|---|---|---|---|---|
| **Base Coat** | | | | | | |
| Polymer of Example 1 | 25 g | 25 g | 20 g | 20 g | 25 g | 25 g |
| Pigmentary paste containing 40% by weight of pigment in isododecane | 5g with DC Red 7 | 5g with DC Red 7 | 5g with DC Red 7 | 5g with DC Red 7 | 5g with red iron oxide | 5g with red iron oxide |
| Disteardimon ium hectorite (Bentone Gel ISD V from Elementis) | 10g | 10g | 10g | 10g | 10g | 10g |
| 2-Octyldodecan ol | | | 20 g | 20 g | | |
| Isododecane | qs 100 g | qs 100 g | qs 100 g | qs 100 g | qs 100 g | qs 100 g |
| **Top Coat** | No | Yes | No | Yes | No | Yes |
| 3-Aminopropyl-terminated polyd imethylsi loxane (Mn 2 500; reference 481688 from Sigma) | | **10g** | | **10g** | | **10g** |
| Isododecane | | 90g | | 90g | | 90g |
| Appearance of the film | Homogene ous film | Homogen eous film | Homogene ous film | Homogen eous film | Homogen eous film | Homogen eous film |
| Water resistance | ++ | +++ | ++ | +++ | ++ | +++ |
| Olive oil resistance | 0 | +++ | 0 | +++ | 0 | +++ |
| Sebum resistance | 0 | +++ | 0 | +++ | 0 | +++ |
| Non-tacky | +++ | +++ | + | +++ | +++ | +++ |
| Transfer-resistant | +++ | +++ | + | +++ | +++ | +++ |

The results obtained show that the deposits resulting from the application of polymer 1 followed by 3-aminopropyl-terminated polydimethylsiloxane (Examples 6, 10) with 2-octyldodecanol (Example 8) form a non-tacky homogeneous film that does not transfer by finger, and that shows better resistance to water, to oil and to sebum, whereas the sole application of polymer 1 (Examples 5, 7, 9) form a deposit that has poor resistance to oil and to sebum. In addition, Example 7 forms a film that is more tacky and that transfers onto the finger.
Thus, the resistance of the film to contact with olive oil and sebum is markedly improved by the application of the top coat composition comprising the 3-aminopropyl-terminated polydimethylsiloxane. Furthermore, when the base coat composition contains 2-octyldodecanol, application of the top coat also makes it possible to improve the tack-free and transfer-resistance properties on contact of the film obtained with a finger.

The lipstick compositions of Examples 6 and 8 applied to the lips thus make it possible to obtain a non-tacky, transfer-resistant and oil- and sebum-resistant makeup which thus has good persistence.

The compositions of Example 10 applied to the skin thus make it possible to obtain a non-tacky, transfer-resistant and oil- and sebum-resistant makeup which thus has good persistence.

### Comparative Examples 11 and 12: Cosmetic evaluation of makeup compositions

The two base coat makeup compositions (lipstick) containing the polymer of Example 2 and a top coat composition containing 3-aminopropyl-terminated polydimethylsiloxane described below were prepared.

The compositions were applied and the cosmetic properties of the film obtained were evaluated as described previously in Examples 5 to 10.

| | Example 11 | Example 12 (invention) |
|---|---|---|
| **Base Coat** | | |
| Polymer of Example 2 | 25 g | 25 g |
| Pigmentary paste containing 40% by weight of pigment in isododecane | 5g with DC Red 7 | 5g with DC Red 7 |
| Disteardimonium hectorite | 10g | 10g |
| (Bentone Gel ISD V from Elementis) | | |
| Isododecane | qs 100 g | qs 100 g |
| **Top Coat** | No | Yes |
| 3-Aminopropyl-terminated polydimethylsiloxane | | **10g** |
| (Mn 2 500; reference 481688 from Sigma) | | |
| Isododecane | | 90g |
| Appearance of the film | Homogeneous film | Homogeneous film |
| Water resistance | ++ | +++ |
| Olive oil resistance | 0 | +++ |
| Sebum resistance | 0 | +++ |
| Non-tacky | +++ | +++ |
| Transfer-resistant | +++ | +++ |

The results obtained show that the deposit resulting from the application of polymer 2 followed by 3-aminopropyl-terminated polydimethylsiloxane (Example 12) forms a non-tacky homogeneous film that does not transfer by finger, and that is resistant to water, to oil and to sebum, whereas the sole application of polymer 2 (Example 11) forms a deposit that has poor resistance to oil and to sebum.
Thus, the resistance of the film to contact with olive oil and sebum is markedly improved by the application of the top coat composition comprising the 3-aminopropyl-terminated polydimethylsiloxane.

The lipstick compositions of Example 12 applied to the lips thus make it possible to obtain a non-tacky, transfer-resistant and water-, oil- and sebum-resistant makeup which thus has good persistence.

### Comparative Examples 13 and 14: Cosmetic evaluation of makeup compositions

The two makeup compositions (foundation) of base coat containing the polymer of Example 3 and a top coat composition containing 3-aminopropyl-terminated polydimethylsiloxane described below were prepared.

The compositions were applied and the cosmetic properties of the film obtained were evaluated as described previously in Examples 5 to 10.

| | Example 13 | Example 14 (invention) |
|---|---|---|
| **Base Coat** | | |
| Polymer of Example 3 | 25 g | 25 g |
| Pigmentary paste containing 40% by weight of pigment in isododecane | 5g with red iron oxide | 5g with red iron oxide |
| Disteardimonium hectorite | 10g | 10g |
| (Bentone Gel ISD V from Elementis) | | |
| Isododecane | qs 100 g | qs 100 g |
| **Top Coat** | No | Yes |
| 3-Aminopropyl-terminated polydimethylsiloxane | | **10g** |
| (Mn 2 500; reference 481688 from Sigma) | | |
| Isododecane | | 90g |
| Appearance of the film | Homogeneous film | Homogeneous film |
| Water resistance | ++ | +++ |
| Olive oil resistance | 0 | +++ |
| Sebum resistance | 0 | +++ |
| Non-tacky | +++ | +++ |
| Transfer-resistant | +++ | +++ |

The results obtained show that the deposit resulting from the application of polymer 3 followed by 3-aminopropyl-terminated polydimethylsiloxane (Example 12) forms a non-tacky homogeneous film that does not transfer by finger, and that is resistant to water, to oil and to sebum, whereas the sole application of polymer 2 (Example 11) forms a deposit that has poor resistance to oil and to sebum.
Thus, the resistance of the film to contact with olive oil and sebum is markedly improved by the application of the top coat composition comprising the 3-aminopropyl-terminated polydimethylsiloxane.

The compositions of Example 14 applied to the skin thus make it possible to obtain a non-tacky, transfer-resistant and water-, oil- and sebum-resistant makeup which thus has good persistence.

### Comparative Examples 15 and 16: Cosmetic evaluation of mascara composition

A base coat composition containing 25% AM of polymer of Example 4, 5% of black iron oxide, 10% of disteardimonium hectorite (Bentone Gel ISD V from Elementis) and 65% of isododecane was applied to a false eyelash specimen. The treated eyelashes were left to dry naturally (25°C) for 24 hours.
0.5 g of a top coat composition containing 10% AM of 3-aminopropyl-terminated polydimethylsiloxane (Mn 2500; reference 481688 from Sigma) in isododecane was then applied to the false eyelashes, and was then left to dry naturally for 24 hours (Example 16 according to the invention).

The persistence with respect to sebum of the deposit formed on the treated eyelashes was then evaluated by immersing the treated eyelashes in artificial sebum for 5 minutes. The eyelashes were then left to dry naturally and were rubbed on blotting paper. No trace of deposit was observed on the paper: the deposit formed on the eyelashes is thus sebum-resistant.
In comparison, the same test was performed on eyelashes treated with the sole application of the base coat composition (without application of the top coat composition) (Example 15): black traces were observed on the blotting paper: the deposit formed on the eyelashes shows poor sebum resistance.

Application of the top coat composition containing the 3-aminopropyl-terminated polydimethylsiloxane makes it possible to improve the sebum resistance of the film obtained.

### Comparative Examples 17 and 18: Cosmetic evaluation of hair composition

0.5 g of a base coat composition containing 10% AM of the polymer of Example 1 in isododecane was applied to a 2.7 g lock of washed and dried hair (lock No. 1). The treated lock was left to dry naturally (25°C) for 24 hours. 0.5 g of a top coat composition containing 10% AM of ethylenediamine in isododecane was then applied to the lock, which was then left to dry naturally for 24 hours (Example 18 according to the invention). The base coat composition alone was applied to another lock of hair (lock No. 2) (Example 17).

The fixing quality of the lock of hair was evaluated by observing the more or less rigid appearance of the lock: the lock is taken by one of its ends with the fingers and turned upside-down, holding it at the bottom; the shape of the lock is then observed; either the lock retains its shape, which means that the lock is fixed very well; or the lock becomes deformed (under the effect of gravity) which means that the lock is not fixed well.

The persistence with respect to water and the fixing property of the treated locks were then evaluated by immersing the treated lock in water for 5 minutes. The locks were then dried manually, followed by drying under a hood. The rigidity of the two locks was observed.

It was found that the lock treated according to Example 18 before and after immersion in water has a rigid form with good hair fixing.
The hair fixing of Example 18 thus shows good persistence with respect to water.
The lock treated according to Example 17 has a rigid form before immersion in the water, but loses its rigidity after immersion in the water: the lock is supple and has no fixing.

### Comparative Examples 19 to 24: Cosmetic evaluation of makeup compositions with application in two steps

The makeup composition (lipstick) of base coat containing the polymer of Example 4 and 5 top coat compositions containing an amine compound chosen from 3-aminopropyl-terminated polydimethylsiloxane (Mn = 25 000 and 50 000), ethylenediamine, polyetherdiamine and bis-cetearyl amodimethicone described below were prepared.

The compositions were applied and the cosmetic properties of the film obtained were evaluated as described previously in Examples 5 to 10.

The following results were obtained:

| | Example 19 | Example 20 (invention) | Example 21 (invention) | Example 22 (invention) | Example 23 (invention) | Example 24 (invention) |
|---|---|---|---|---|---|---|
| **Base Coat** | | | | | | |
| Polymer of Example 4 | 20 g AM | 20 g AM | 20 g AM | 20 g AM | 20 g AM | 20 g AM |
| Pigmentary paste containing 40% by weight of pigment in isododecane | 5 g with DC Red 7 | 5 g with DC Red 7 | 5 g with DC Red 7 | 5 g with DC Red 7 | 5 g with DC Red 7 | 5 g with DC Red 7 |
| Disteardimonium hectorite | 10 g | 10 g | 10 g | 10 g | 10 g | 10 g |
| (Bentone Gel ISD V from Elementis) | | | | | | |
| Isododecane | qs 100 g | qs 100 g | qs 100 g | qs 100 g | qs 100 g | qs 100 g |

| **Top Coat** | | | | | | |
|---|---|---|---|---|---|---|
| 3-Aminopropyl-terminated polydimethylsiloxa ne | | **10 g** | | | | |
| (Mn 25 000; DMS A-31 from Gelest) | | | | | | |
| 3-Aminopropyl-terminated polydimethylsiloxa ne | | | **10 g** | | | |
| (Mn 25 000; DMS A-35 from Gelest) | | | | | | |
| Ethylenediamine | | | | **10 g** | | |
| Polyetherdiamine (1) | | | | | **10 g** | |
| Bis-cetearyl amodimethicone (2) | | | | | | **10 g** |
| Isododecane | | qs 100 g | qs 100 g | qs 100 g | qs 100 g | qs 100 g |

| **Evaluation of the film** | | | | | | |
|---|---|---|---|---|---|---|
| Appearance of the film | Homogeneous film | Homogeneous film | Homogeneous film | Homogeneous film | Homogeneous film | Homogeneous film |
| Olive oil resistance | 0 | +++ | ++ | ++ | ++ | ++ |
| Non-tacky | +++ | +++ | +++ | +++ | +++ | +++ |
| Transfer-resistant | ++ | +++ | +++ | +++ | +++ | +++ |

| | | | | | | |
|---|---|---|---|---|---|---|
| (1) Jeffamine® ED-900 Polyetheramine (Huntsman) (2) Silsoft® AX (Momentive Performance Materials) | | | | | | |

The results obtained show that the deposit resulting from the application of polymer 4 followed by the amine compound (Examples 20 to 24) forms a non-tacky homogeneous film that does not transfer by finger, and that is resistant to oil, whereas the sole application of polymer 4 (Example 19) forms a deposit that transfers onto the finger and has poor resistance to oil.
Thus, the non-tacky and transfer-resistant aspect on contact with the finger, and also the resistance of the film to contact with olive oil are improved with the application of the top coat composition containing the amine compounds tested.

The compositions of Examples 20 to 24 applied to the lips thus make it possible to obtain a non-tacky, transfer-resistant and oil-resistant makeup which thus has good persistence.

## Claims

1. Cosmetic process for treating keratin materials, comprising the sequential application to the keratin materials of a cosmetic composition comprising a maleic anhydride acrylic polymer and of a polyamine compound or a cosmetic composition containing same,
said maleic anhydride acrylic polymer being able to be obtained by polymerization of:
(a) 50% to 90% by weight, relative to the total weight of monomers, of isobornyl (meth)acrylate
(b) 1 % to 50% by weight of maleic anhydride
(c) 0 to 49% by weight of additional (meth)acrylate monomer chosen from:
(i) linear or branched, saturated or unsaturated C₁-C₂₀ alkyl (meth)acrylates, optionally interrupted with one or more non-adjacent heteroatoms chosen from O and S or with a group NR, R being a C₁-C₄ alkyl group, optionally substituted with a phenyl or furfuryl group;
(ii) saturated C₄-C₈ cycloalkyl (meth)acrylates optionally interrupted with O or NH;
said polyamine compound bearing several primary amine and/or secondary amine groups, the polyamine compound not being an alkoxysilane.

2. Process according to Claim 1, **characterized in that** said maleic anhydride acrylic polymer is derived from the polymerization of:
(a) 50% to 80% by weight, relative to the total weight of monomers, of isobornyl (meth)acrylate
(b) 5% to 30% by weight of maleic anhydride
(c) 15% to 30% by weight of said additional (meth)acrylate monomer.

3. Process according to either of the preceding claims, **characterized in that** said maleic anhydride acrylic polymer is derived from the polymerization of:
(a) 50% to 80% by weight, relative to the total weight of monomers, of isobornyl (meth)acrylate
(b) 5% to 25% by weight of maleic anhydride
(c) 15% to 30% by weight of said additional (meth)acrylate monomer.

4. Process according to one of the preceding claims, **characterized in that** the maleic anhydride acrylic polymer is derived from the polymerization of:
(a) 50% to 80% by weight, relative to the total weight of monomers, of isobornyl (meth)acrylate
(b) 5% to 15% by weight of maleic anhydride
(c) 15% to 30% by weight of said additional (meth)acrylate monomer.

5. Process according to one of the preceding claims, **characterized in that** the maleic anhydride acrylic polymer is derived from the polymerization of:
(a) 60% to 80% by weight, relative to the total weight of monomers, of isobornyl (meth)acrylate
(b) 5% to 12% by weight of maleic anhydride
(c) 15% to 30% by weight of said additional (meth)acrylate monomer.

6. Process according to one of the preceding claims, **characterized in that** said additional (meth)acrylate monomer is chosen from C₆-C₁₆ alkyl (meth)acrylates and preferably from C₆-C₁₆ alkyl acrylates.

7. Process according to one of the preceding claims, **characterized in that** said maleic anhydride acrylic polymer is derived from the polymerization of:
(a) 50% to 80% by weight, relative to the total weight of monomers, of isobornyl (meth)acrylate
(b) 5% to 30% by weight of maleic anhydride
(c) 15% to 30% by weight of C₆-C₁₆ alkyl acrylate monomer.

8. Process according to one of the preceding claims, **characterized in that** the maleic anhydride acrylic polymer is derived from the polymerization of:
(a) 50% to 80% by weight, relative to the total weight of monomers, of isobornyl (meth)acrylate
(b) 5% to 25% by weight of maleic anhydride
(c) 15% to 30% by weight of C₆-C₁₆ alkyl acrylate monomer
and **in that** it is preferably derived from the polymerization of:
(a) 50% to 80% by weight, relative to the total weight of monomers, of isobornyl (meth)acrylate
(b) 5% to 15% by weight of maleic anhydride
(c) 15% to 30% by weight of C₆-C₁₆ alkyl acrylate monomer.

9. Process according to one of the preceding claims, **characterized in that** the acrylic polymer comprises isobornyl acrylate, 2-ethylhexyl acrylate and maleic anhydride.

10. Process according to any one of the preceding claims, **characterized in that** the acrylic polymer has a weight-average molecular weight ranging from 5000 to 1 000 000 g/mol, preferably ranging from 10 000 to 500 000 g/mol, preferentially ranging from 15 000 to 350 000 g/mol.

11. Process according to any one of the preceding claims, **characterized in that** the polyamine compound comprises from 2 to 20 carbon atoms.

12. Process according to any one of the preceding claims, **characterized in that** the polyamine compound is chosen from N-methyl-1,3-diaminopropane, N-propyl-1,3-diaminopropane, N-isopropyl-1,3-diaminopropane, N-cyclohexyl-1,3-diaminopropane, 2-(3-aminopropylamino)ethanol, 3-(2-aminoethyl)aminopropylamine, bis(3-aminopropyl)amine, methylbis(3-aminopropyl)amine, N-(3-aminopropyl)-1,4-diaminobutane, N,N-dimethyldipropylenetriamine, 1,2-bis(3-aminopropylamino)ethane, N,N'-bis(3-aminopropyl)-1,3-propanediamine, ethylenediamine, 1,3-propylenedimaine, 1,4-butylenediamine, lysine, cystamine, xylenediamine, tris(2-aminoethyl)amine and spermidine;
preferably from ethylenediamine, 1,3-propylenediamine and 1,4-butylenediamine; it is preferentially ethylenediamine.

13. Process according to one of Claims 1 to 10, **characterized in that** the polyamine compound is chosen from amine-based polymers, especially having a weight-average molecular weight ranging from 500 to 1 000 000, preferably ranging from 500 to 500 000, and preferentially ranging from 500 to 100 000.

14. Process according to the preceding claim, **characterized in that** the polyamine compound is chosen from poly((C₂-C₅)alkyleneimines), and in particular polyethyleneimines and polypropyleneimines, especially poly(ethyleneimine)s; poly(allylamine); polyvinylamines and copolymers thereof, in particular with vinylamides; vinylamine/vinylformamide copolymers; polyamino acids bearing NH₂ groups, such as polylysine; aminodextran; amino polyvinyl alcohol, acrylamidopropylamine-based copolymers; chitosans;
polydimethylsiloxanes comprising primary amine groups at the chain end or on side chains, for example aminopropyl side or end groups, for instance those of formula (A) or (B) or (C): 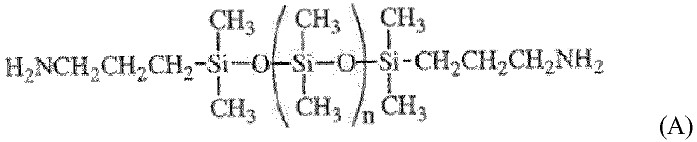 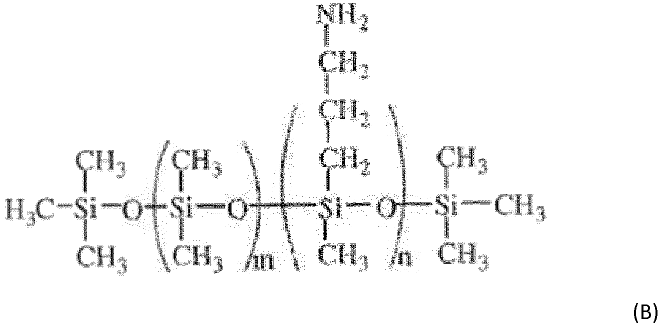
H₂NCH₂CH₂CH₂Si(CH₃)₂O-[Si(CH₃)₂-O]ₙ-Si(CH₃)₂C₄H₉ (C)
with:
in formula (A): the value of n is such that the weight-average molecular weight of the silicone is between 500 and 55 000;
in formula (B), the values of n and m are such that the weight-average molecular weight of the silicone is between 1000 and 55 000;
in formula (C), the value of n is such that the weight-average molecular weight of the silicone is between 500 and 3000;
amodimethicones of formula (D): 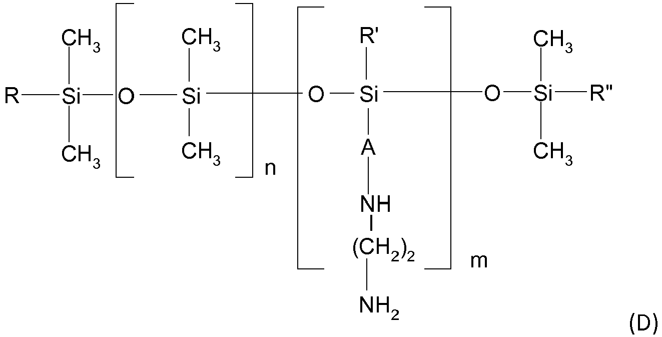 in which R, R' and R", which may be identical or different, each represent a C₁-C₄ alkyl or hydroxyl group, A represents a C₃ alkylene group and m and n are such that the weight-average molecular mass of the compound is between 5000 and 500 000 approximately;
amodimethicones of formula (K): 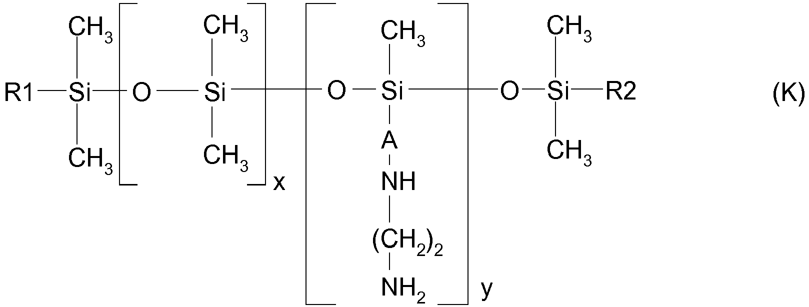 in which:
- R1 and R2, which may be identical or different, preferably identical, represent a linear or branched, saturated or unsaturated alkyl group comprising from 6 to 30 carbon atoms, preferably from 8 to 24 carbon atoms and preferentially from 12 to 20 carbon atoms,
- A represents a linear or branched alkylene radical group containing from 2 to 8 carbon atoms,
- x and y are integers ranging from 1 to 5000; preferably, x ranges from 10 to 2000 and especially from 100 to 1000; preferably, y ranges from 1 to 100;
preferentially, for the amodimethicone of formula (K):
- x ranges from 10 to 2000 and especially from 100 to 1000;
- y ranges from 1 to 100;
- A comprises from 3 to 6 carbon atoms, and in particular 4 carbon atoms; preferably, A is branched; preferentially, A is chosen from the divalent radicals: -CH₂CH₂CH₂- and
- CH₂CH(CH₃)CH₂-; and
- R1 and R2, which may be identical or different, represent a saturated linear radical comprising from 6 to 30 carbon atoms, preferably from 8 to 24 carbon atoms and especially from 12 to 20 carbon atoms, for instance a dodecyl, tetradecyl, pentadecyl, hexadecyl, heptadecyl, octadecyl, nonadecyl or eicosyl group; advantageously, R1 and R2 represent a mixture of hexadecyl (cetyl) and octadecyl (stearyl) radicals (mixture also known as cetearyl);
preferably, the amodimethicone of formula (K) is bis-cetearyl amodimethicone;
polyetherdiamines and especially polyethylene glycol and/or polypropylene glycol α,ω-diamines; polytetrahydrofuran (or polytetramethylene glycol) α,ω-diamines and polybutadiene α,ω-diamines;
polyamidoamine dendrimers bearing amine end functions;
poly(meth)acrylates or poly(meth)acrylamides bearing primary or secondary amine side functions, such as poly(3-aminopropyl)methacrylamide or poly(2-aminoethyl) methacrylate;
preferably polydimethylsiloxanes comprising primary amine groups at the chain end or on side chains; amodimethicones of formula (K); polyethylene glycol and/or polypropylene glycol α,ω-diamines;ethylenediamine, 1,3-propylenediamine and 1,4-butylenediamine;
preferentially, polydimethylsiloxanes comprising aminopropyl end groups at the chain end, bis-cetearyl amodimethicone, polyethylene glycol/polypropylene glycol α,ω-diamine copolymers comprising from 2 to 50 units derived from ethylene oxide and from 1 to 10 units derived from propylene oxide.

15. Process according to any one of the preceding claims, **characterized in that** the polyamine compound is used in a mole ratio of the amine group of the polyamine compound/maleic anhydride group of the acrylic polymer ranging from 0.01 to 10, preferably ranging from 0.1 to 5, preferentially ranging from 0.1 to 2 and more preferentially ranging from 0.1 to 1.

16. Process according to any one of the preceding claims, **characterized in that** the acrylic polymer is present in a content ranging from 0.1% to 40% by weight, relative to the total weight of the composition, preferably from 0.5% to 35% by weight of active material, preferentially ranging from 1% to 30% by weight, and more preferentially ranging from 10% to 30% by weight.

17. Process according to any one of the preceding claims, **characterized in that** the composition comprises a hydrocarbon-based oil, preferably an apolar hydrocarbon-based oil containing from 8 to 14 carbon atoms, preferentially isododecane.

18. Process according to any one of Claims 1 to 17, **characterized in that** the composition comprising the maleic anhydride acrylic polymer is first applied to the keratin materials, and the polyamine compound or a composition containing same and comprising a physiologically acceptable medium is then applied.

19. Process according to any one of Claims 1 to 17, **characterized in that** the amine compound, or a composition containing same and comprising a physiologically acceptable medium, is first applied to the keratin materials, and the composition comprising the maleic anhydride acrylic polymer is then applied.

20. Process according to any one of the preceding claims, **characterized in that** it is performed on the skin, the lips, the eyelashes, the hair or the nails.

21. Kit comprising a first composition comprising a maleic anhydride acrylic polymer as defined in any one of Claims 1 to 10 and comprising a physiologically acceptable medium, and a second composition comprising a polyamine compound as defined in one of Claims 1 and 11 to 14 and comprising a physiologically acceptable medium, the first and second compositions each being packaged in a separate packaging assembly.

22. Polymer that may be obtained by reacting a maleic anhydride acrylic polymer defined in any one of Claims 1 to 10 with a polyamine compound as defined in one of Claims 1 and 11 to 14.

## Patentansprüche

1. Kosmetisches Verfahren zur Behandlung von Keratinmaterialien, umfassend das aufeinanderfolgende Aufbringen einer kosmetischen Zusammensetzung, die ein Maleinsäureanhydrid-Acrylpolymer umfasst, und einer Polyaminverbindung oder einer kosmetischen Zusammensetzung, die diese enthält, auf die Keratinmaterialien,
wobei das Maleinsäureanhydrid-Acrylpolymer durch Polymerisation von:
(a) 50 bis 90 Gew.-%, bezogen auf das Gesamtgewicht der Monomere, Isobornyl(meth)acrylat,
(b) 1 bis 50 Gew.-% Maleinsäureanhydrid,
(c) 0 bis 49 Gew.-%zusätzlichem (Meth)acrylat-Monomer, ausgewählt aus:
(i) linearen oder verzweigten, gesättigten oder ungesättigten C₁-C₂₀-Alkyl (meth) acrylaten, die gegebenenfalls durch ein oder mehrere nicht benachbarte Heteroatome, die aus O und S ausgewählt sind, oder durch eine Gruppe NR unterbrochen sind, wobei R für eine C₁-C₄-Alkylgruppe, die gegebenenfalls durch eine Phenyl- oder Furfurylgruppe substituiert ist, steht;
(ii) gesättigten C₄-C₈-Cycloalkyl (meth) acrylaten, die gegebenenfalls durch O oder NH unterbrochen sind;
erhältlich ist;
wobei die Polyaminverbindung mehrere primäre Amin-und/oder sekundäre Amingruppen trägt, wobei es sich bei der Polyaminverbindung nicht um ein Alkoxysilan handelt.

2. Verfahren nach Anspruch 1, **dadurch gekennzeichnet, dass** sich das Maleinsäureanhydrid-Acrylpolymer aus der Polymerisation von:
(a) 50 bis 80 Gew.-%, bezogen auf das Gesamtgewicht der Monomere, Isobornyl(meth)acrylat,
(b) 5 bis 30 Gew.-% Maleinsäureanhydrid,
(c) 15 bis 30 Gew.-% des zusätzlichen (Meth)-acrylat-Monomers
ableitet.

3. Verfahren nach einem der vorhergehenden Ansprüche, **dadurch gekennzeichnet, dass** sich das Maleinsäureanhydrid-Acrylpolymer aus der Polymerisation von:
(a) 50 bis 80 Gew.-%, bezogen auf das Gesamtgewicht der Monomere, Isobornyl(meth)acrylat,
(b) 5 bis 25 Gew. - % Maleinsäureanhydrid,
(c) 15 bis 30 Gew.-% des zusätzlichen (Meth)-acrylat-Monomers
ableitet.

4. Verfahren nach einem der vorhergehenden Ansprüche, **dadurch gekennzeichnet, dass** sich das Maleinsäureanhydrid-Acrylpolymer aus der Polymerisation von:
(a) 50 bis 80 Gew.-%, bezogen auf das Gesamtgewicht der Monomere, Isobornyl(meth)acrylat,
(b) 5 bis 15 Gew.-% Maleinsäureanhydrid,
(c) 15 bis 30 Gew.-% des zusätzlichen (Meth)-acrylat-Monomers
ableitet.

5. Verfahren nach einem der vorhergehenden Ansprüche, **dadurch gekennzeichnet, dass** sich das Maleinsäureanhydrid-Acrylpolymer aus der Polymerisation von:
(a) 60 bis 80 Gew.-%, bezogen auf das Gesamtgewicht der Monomere, Isobornyl(meth)acrylat,
(b) 5 bis 12 Gew.-% Maleinsäureanhydrid,
(c) 15 bis 30 Gew.-% des zusätzlichen (Meth)-acrylat-Monomers
ableitet.

6. Verfahren nach einem der vorhergehenden Ansprüche, **dadurch gekennzeichnet, dass** das zusätzliche (Meth)acrylat-Monomer aus C₆-C-₁₆-Alkyl(meth)-acrylaten und vorzugsweise aus C₆-C₁₆-Alkylacrylaten ausgewählt ist.

7. Verfahren nach einem der vorhergehenden Ansprüche, **dadurch gekennzeichnet, dass** sich das Maleinsäureanhydrid-Acrylpolymer aus der Polymerisation von:
(a) 50 bis 80 Gew.-%, bezogen auf das Gesamtgewicht der Monomere, Isobornyl(meth)acrylat,
(b) 5 bis 30 Gew.-% Maleinsäureanhydrid,
(c) 15 bis 30 Gew.-% C₆-C₁₆-Alkylacrylat-Monomer ableitet.

8. Verfahren nach einem der vorhergehenden Ansprüche, **dadurch gekennzeichnet, dass** sich das Maleinsäureanhydrid-Acrylpolymer aus der Polymerisation von:
(a) 50 bis 80 Gew.-%, bezogen auf das Gesamtgewicht der Monomere, Isobornyl(meth)acrylat,
(b) 5 bis 25 Gew.-% Maleinsäureanhydrid,
(c) 15 bis 30 Gew.-% C₆-C₁₆-Alkylacrylat-Monomer ableitet
und sich vorzugsweise aus der Polymerisation von:
(a) 50 bis 80 Gew.-%, bezogen auf das Gesamtgewicht der Monomere, Isobornyl(meth)acrylat,
(b) 5 bis 15 Gew.-% Maleinsäureanhydrid,
(c) 15 bis 30 Gew.-% C₆-C₁₆-Alkylacrylat-Monomer ableitet.

9. Verfahren nach einem der vorhergehenden Ansprüche, **dadurch gekennzeichnet, dass** das Acrylpolymer Isobornylacrylat, 2-Ethylhexylacrylat und Maleinsäureanhydrid umfasst.

10. Verfahren nach einem der vorhergehenden Ansprüche, **dadurch gekennzeichnet, dass** das Acrylpolymer ein gewichtsmittleres Molekulargewicht im Bereich von 5000 bis 1.000.000 g/mol, vorzugsweise im Bereich von 10.000 bis 500.000 g/mol und bevorzugt im Bereich von 15.000 bis 350.000 g/mol aufweist.

11. Verfahren nach einem der vorhergehenden Ansprüche, **dadurch gekennzeichnet, dass** die Polyaminverbindung 2 bis 20 Kohlenstoffatome umfasst.

12. Verfahren nach einem der vorhergehenden Ansprüche, **dadurch gekennzeichnet, dass** die Polyaminverbindung aus N-Methyl-1,3-diaminopropan, N-Propyl-1,3-diaminopropan, N-Isopropyl-1,3-diaminopropan, N-Cyclohexyl-1,3-diaminopropan, 2-(3-Aminopropylamino)ethanol, 3-(2-Aminoethyl)aminopropylamin, Bis(3-aminopropyl)amin, Methylbis(3-aminopropyl)-amin, N-(3-Aminopropyl)-1,4-diaminobutan, N,N-Dimethyldipropylentriamin, 1,2-Bis(3-aminopropylamino)ethan, N,N'-Bis(3-aminopropyl)-1,3-propan-diamin, Ethylendiamin, 1,3-Propylendiamin, 1,4-Butylendiamin, Lysin, Cystamin, Xyloldiamin, Tris(2-aminoethyl)amin und Spermidin und vorzugsweise aus Ethylendiamin, 1,3-Propylendiamin und 1,4-Butylendiamin ausgewählt ist und bevorzugt Ethylendiamin ist.

13. Verfahren nach einem der Ansprüche 1 bis 10, **dadurch gekennzeichnet, dass** die Polyaminverbindung aus Polymeren auf Amin-Basis, insbesondere mit einem gewichtsmittleren Molekulargewicht im Bereich von 500 bis 1.000.000, vorzugsweise im Bereich von 500 bis 500.000 und bevorzugt im Bereich von 500 bis 100.000 ausgewählt ist.

14. Verfahren nach dem vorhergehenden Anspruch, **dadurch gekennzeichnet, dass** die Polyaminverbindung aus Poly((C₂-C₅)alkyleniminen) und insbesondere Polyethyleniminen und Polypropyleniminen, insbesondere Poly(ethylenimin)en; Poly-(allylamin); Polyvinylaminen und Copolymeren davon, insbesondere mit Vinylamiden; Vinylamin/Vinylformamid-Copolymeren; Polyaminosäuren mit NH₂-Gruppen, wie Polylysin; Aminodextran; Aminopolyvinylalkohol, Copolymeren auf Basis von Acrylamidopropylamin; Chitosanen; Polydimethylsiloxanen mit primären Amingruppen am Kettenende oder an Seitenketten, beispielsweise Aminopropyl-Endgruppen oder -Seitengruppen, wie beispielsweise denjenigen der Formel (A) oder (B) oder (C): 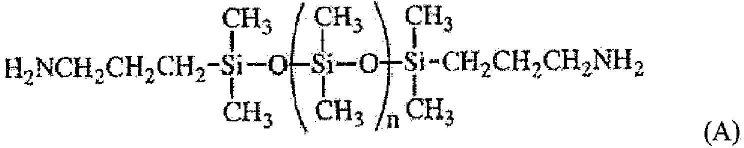 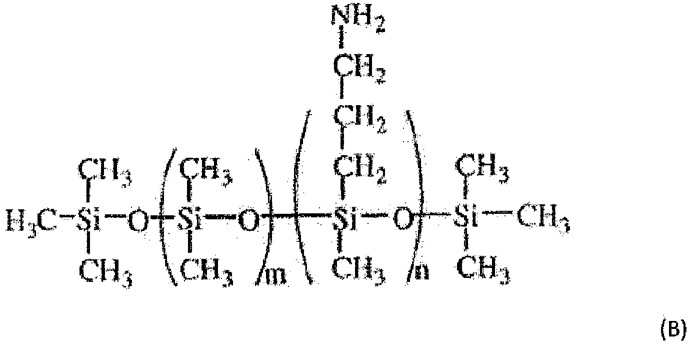
H₂NCH₂CH₂CH₂-Si(CH₃)₂-O- [Si (CH₃)₂-O]ₙ-Si(CH₃)₂C₄H₉ (C)
wobei:
in der Formel (A) : der Wert von n so beschaffen ist, dass das gewichtsmittlere Molekulargewicht des Silikons zwischen 500 und 55.000 liegt;
in der Formel (B) die Werte von n und m so beschaffen sind, dass das gewichtsmittlere Molekulargewicht des Silikons zwischen 1000 und 55.000 liegt;
in der Formel (C) der Wert von n so beschaffen ist, dass das gewichtsmittlere Molekulargewicht des Silikons zwischen 500 und 3000 liegt;
Amodimethiconen der Formel (D): 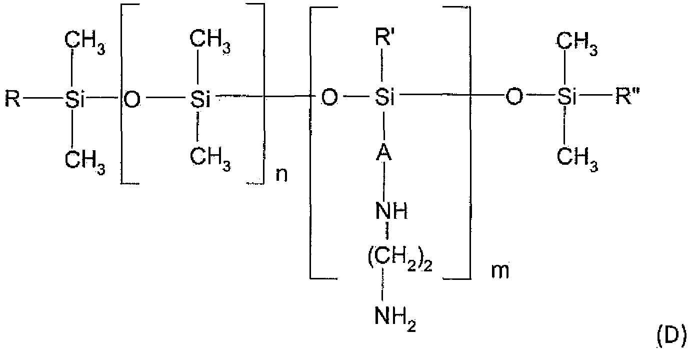 in der R, R' und R'', die gleich oder verschieden sein können, jeweils für eine C₁-C₄-Alkyl- oder Hydroxylgruppe stehen, A für eine C₃-Alkylengruppe steht und m und n so beschaffen sind, dass das gewichtsmittlere Molekulargewicht der Verbindung zwischen ungefähr 5000 und 500.000 liegt; Amodimethiconen der Formel (K): 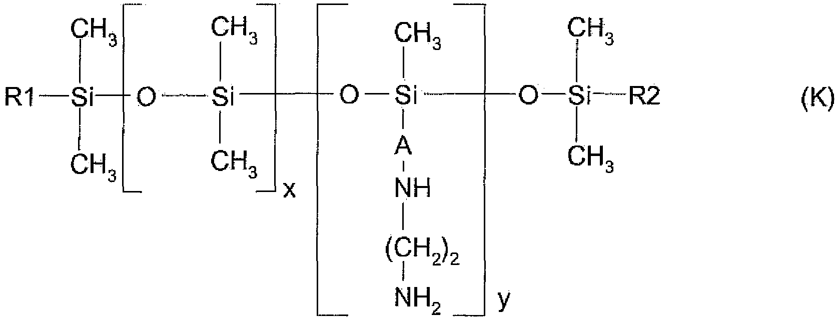 in der :
- R1 und R2, die gleich oder verschieden, vorzugsweise gleich, sein können, für eine lineare öder verzweigte, gesättigte oder ungesättigte Alkylgruppe mit 6 bis 30 Kohlenstoffatomen, vorzugsweise 8 bis 24 Kohlenstoffatomen und bevorzugt 12 bis 20 Kohlenstoffatomen stehen,
- für eine lineare oder verzweigte Alkylenrestgruppe mit 2 bis 8 Kohlenstoffatomen steht,
- x und y ganze Zahlen im Bereich von 1 bis 5000 sind, x vorzugsweise im Bereich von 10 bis 2000 und insbesondere von 100 bis 1000 liegt, y vorzugsweise im Bereich von 1 bis 100 liegt;
bevorzugt für das Amodimethicon der Formel (K):
- x im Bereich von 10 bis 2000 und insbesondere von 100 bis 1000 liegt;
- y im Bereich von 1 bis 100 liegt;
- A 3 bis 6 Kohlenstoffatome und insbesondere 4 Kohlenstoffatome umfasst; vorzugsweise A verzweigt ist; bevorzugt A aus den zweiwertigen Resten -CH₂CH₂CH₂- und -CH₂CH(CH₃)CH₂- ausgewählt ist; und
- R1 und R2, die gleich oder verschieden sind, für einen gesättigten linearen Rest mit 6 bis 30 Kohlenstoffatomen, vorzugsweise 8 bis 24 Kohlenstoffatomen und insbesondere 12 bis 20 Kohlenstoffatomen, beispielsweise eine Dodecyl-, Tetradecyl-, Pentadecyl-, Hexadecyl-, Heptadecyl-, Octadecyl-, Nonadecyl- oder Eicosylgruppe, stehen; vorteilhafterweise R1 und R2 für eine Mischung von Hexadecyl(Cetyl)- und Octadecyl(Stearyl)-Resten stehen, wobei die Mischung auch als Cetearyl bekannt ist);
es sich vorzugsweise bei dem Amodimethicon der Formel (K) um Biscetearylamodimethicon handelt; Polyetherdiaminen und insbesondere Polyethylenglykol- und/oder Polypropylenglykol-α,ω-diaminen; Polytetrahydrofuran-α,ω-diaminen (oder Polytetramethylenglykol-α,ω-diaminen) und Polybutadien-α,ω-diaminen;
Polyamidoamin-Dendrimeren mit endständigen Aminfunktionen;
Poly(meth)acrylaten oder Poly(meth)acrylamiden mit seitenständigen primären oder sekundären Aminfunktionen wie Poly(3-aminopropyl)methacrylamid oder Poly(2-aminoethyl)methacrylat;
vorzugsweise Polydimethylsiloxanen mit primären Amingruppen am Kettenende oder an Seitenketten; Amodimethiconen der Formel (K) ; Polyethylenglykol-und/oder Polypropylenglykol-α,ω-diaminen; Ethylendiamin, 1,3-Propylendiamin und 1,4-Butylendiamin;
bevorzugt Polydimethylsiloxanen mit Aminopropyl-Endgruppen am Kettenende, Biscetearylamodimethicon, Polyethylenglykol-/Polypropylenglykol-α,ω-diamin-Copolymeren mit 2 bis 50 von Ethylenoxid abgeleiteten Einheiten und 1 bis 10 von Propylenoxid abgeleiteten Einheiten; ausgewählt ist.

15. Verfahren nach einem der vorhergehenden Ansprüche, **dadurch gekennzeichnet, dass** die Polyaminverbindung in einem Molverhältnis von Aminogruppe der Polyaminverbindung zu Maleinsäureanhydridgruppe des Acrylpolymers im Bereich von 0,01 bis 10, vorzugsweise im Bereich von 0,1 bis 5, bevorzugt im Bereich von 0,1 bis 2 und weiter bevorzugt im Bereich von 0,1 bis 1 verwendet wird.

16. Verfahren nach einem der vorhergehenden Ansprüche, **dadurch gekennzeichnet, dass** das Acrylpolymer in einem Gehalt im Bereich von 0,1 bis 40 Gew.-%, bezogen auf das Gesamtgewicht der Zusammensetzung, vorzugsweise von 0,5 bis 35 Gew.-% Wirkstoff, bevorzugt im Bereich von 1 bis 30 Gew.-% und weiter bevorzugt im Bereich von 10 bis 30 Gew.-% vorliegt.

17. Verfahren nach einem der vorhergehenden Ansprüche, **dadurch gekennzeichnet, dass** die Zusammensetzung ein Öl auf Kohlenwasserstoffbasis, vorzugsweise ein unpolares Öl auf Kohlenwasserstoffbasis mit 8 bis 14 Kohlenstoffatomen, bevorzugt Isododecan, umfasst.

18. Verfahren nach einem der Ansprüche 1 bis 17, **dadurch gekennzeichnet, dass** man auf die Keratinmaterialien zunächst die Zusammensetzung, die das Maleinsäureanhydrid-Acrylpolymer umfasst, aufbringt und dann die Polyaminverbindung oder eine Zusammensetzung, die diese enthält und ein physiologisch unbedenkliches Medium umfasst, aufbringt.

19. Verfahren nach einem der Ansprüche 1 bis 17, **dadurch gekennzeichnet, dass** man auf die Keratinmaterialien zunächst die Aminverbindung oder eine Zusammensetzung, die diese enthält und ein physiologisch unbedenklich Medium umfasst, aufbringt und dann die Zusammensetzung, die das Maleinsäureanhydrid-Acrylpolymer umfasst, aufbringt.

20. Verfahren nach einem der vorhergehenden Ansprüche, **dadurch gekennzeichnet, dass** es auf die Haut, die Lippen, die Wimpern, das Haar oder die Nägel angewendet wird.

21. Kit, umfassend eine erste Zusammensetzung, die ein Maleinsäureanhydrid-Acrylpolymer gemäß einem der Ansprüche 1 bis 10 und ein physiologisch unbedenkliches Medium umfasst, und eine zweite Zusammensetzung, die eine Polyaminverbindung gemäß einem der Ansprüche 1 und 11 bis 14 und ein physiologisch unbedenkliches Medium umfasst, wobei die erste Zusammensetzung und die zweite Zusammensetzung jeweils in einer separaten Verpackungsanordnung verpackt sind.

22. Polymer, das durch Umsetzung eines Maleinsäureanhydrid-Acrylpolymers gemäß einem der Ansprüche 1 bis 10 mit einer Polyaminverbindung gemäß einem der Ansprüche 1 und 11 bis 14 erhältlich ist.

## Revendications

1. Procédé cosmétique pour le traitement de matières kératiniques comprenant l'application séquentielle sur les matières kératiniques d'une composition cosmétique comprenant un polymère acrylique d'anhydride maléique et d'un composé polyamine ou d'une composition cosmétique le contenant,
ledit polymère acrylique d'anhydride maléique étant susceptible d'être obtenu par polymérisation de :
(a) 50 à 90 % en poids, du poids total de monomères, de (méth)acrylate d'isobornyle
(b) 1 à 50 % en poids d'anhydride maléique
(c) 0 à 49 % en poids de monomère (méth)acrylate additionnel choisi parmi :
(i) les (méth)acrylates d'alkyle en C₁-C₂₀, linéaire ou ramifié, saturé ou insaturé, éventuellement interrompus par un ou plusieurs hétéroatomes non adjacents choisis parmi O ou S ou par un groupe NR, R étant un groupe alkyle en C₁-C₄, éventuellement substitué par un groupe phényle ou furfuryle ;
(ii) les (méth) acrylates de cycloalkyle en C₄-C₈ saturé éventuellement interrompus par O ou NH ;
ledit composé polyamine étant porteur de plusieurs groupes amine primaire et/ou amine secondaire, le composé polyamine n'étant pas un alcoxy silane.

2. Procédé selon la revendication 1, **caractérisé en ce que** ledit polymère acrylique d'anhydride maléique est issu de la polymérisation de :
(a) 50 à 80 % en poids, du poids total de monomères, de (méth)acrylate d'isobornyle
(b) 5 à 30 % en poids d'anhydride maléique
(c) 15 à 30 % en poids dudit monomère (méth)acrylate additionnel.

3. Procédé selon l'une ou l'autre des revendications précédentes, **caractérisé en ce que** ledit polymère acrylique d'anhydride maléique est issu de la polymérisation de :
(a) 50 à 80 % en poids, du poids total de monomères, de (méth)acrylate d'isobornyle
(b) 5 à 25 % en poids d'anhydride maléique
(c) 15 à 30 % en poids dudit monomère (méth)acrylate additionnel.

4. Procédé selon l'une des revendications précédentes, **caractérisé en ce que** le polymère acrylique d'anhydride maléique est issu de la polymérisation de :
(a) 50 à 80 % en poids, du poids total de monomères, de (méth)acrylate d'isobornyle
(b) 5 à 15 % en poids d'anhydride maléique
(c) 15 à 30 % en poids dudit monomère (méth)acrylate additionnel.

5. Procédé selon l'une des revendications précédentes, **caractérisé en ce que** le polymère acrylique d'anhydride maléique est issu de la polymérisation de :
(a) 60 à 80 % en poids, du poids total de monomères, de (méth)acrylate d'isobornyle
(b) 5 à 12 % en poids d'anhydride maléique
(c) 15 à 30 % en poids dudit monomère (méth) acrylate additionnel.

6. Procédé selon l'une des revendications précédentes, **caractérisé en ce que** ledit monomère (méth)acrylate additionnel est choisi parmi les (méth)acrylates d'alkyle en C6-C16, et de préférence parmi les acrylates d'alkyle en C6-C16.

7. Procédé selon l'une des revendications précédentes, **caractérisé en ce que** ledit polymère acrylique d'anhydride maléique est issu de la polymérisation de :
(a) 50 à 80 % en poids, du poids total de monomères, de (méth)acrylate d'isobornyle
(b) 5 à 30 % en poids d'anhydride maléique
(c) 15 à 30 % en poids de monomère acrylate d'alkyle en C6-C16.

8. Procédé selon l'une des revendications précédentes, **caractérisé en ce que** le polymère acrylique d'anhydride maléique est issu de la polymérisation de :
(a) 50 à 80 % en poids, du poids total de monomères, de (méth)acrylate d'isobornyle
(b) 5 à 25 % en poids d'anhydride maléique
(c) 15 à 30 % en poids de monomère acrylate d'alkyle en C6-C16,
et **en ce qu'**il est de préférence issu de la polymérisation de :
(a) 50 à 80 % en poids, du poids total de monomères, de (méth)acrylate d'isobornyle
(b) 5 à 15 % en poids d'anhydride maléique
(c) 15 à 30 % en poids de monomère acrylate d'alkyle en C6-C16.

9. Procédé selon l'une des revendications précédentes, **caractérisé en ce que** le polymère acrylique comprend de l'acrylate d'isobornyle, de l'acrylate de 2-éthylhexyle et de l'anhydride maléique.

10. Procédé selon l'une quelconque des revendications précédentes, **caractérisé en ce que** le polymère acrylique a un poids moléculaire moyen en poids allant de 5000 à 1 000 000 g/mole, de préférence allant de 10 000 à 500 000 g/mole, et préférentiellement allant de 15 000 à 350 000 g/mole.

11. Procédé selon l'une quelconque des revendications précédentes, **caractérisé en ce que** le composé polyamine comprend de 2 à 20 atomes de carbone.

12. Procédé selon l'une quelconque des revendications précédentes, **caractérisé en ce que** le composé polyamine est choisi parmi le N-méthyl-1,3-diaminopropane, le N-propyl-1,3-diaminopropane, le N-isopropyl-1,3-diaminopropane, le N-cyclohexyl-1,3-diaminopropane, le 2- (3-aminopropylamino) éthanol, la 3-(2-aminoéthyl)aminopropylamine, la bis (3-aminopropyl) amine, la méthylbis(3-aminopropyl)amine, le N-(3-aminopropyl)-1,4-diaminobutane, la N,N-diméthyldipropylènetriamine, le 1,2-bis(3-aminopropylamino)éthane, la N,N'-bis(3-aminopropyl)-1, 3-propanediamine, l'éthylène diamine, la 1,3-propylènediamine, la 1,4-butylènediamine, la lysine, la cystéamine , la xylène diamine, la tris(2-aminoéthyl)amine et la spermidine ;
de préférence parmi l'éthylène diamine, la 1,3-propylènediamine et la 1,4-butylènediamine ; il est préférentiellement l'éthylène diamine.

13. Procédé selon l'une des revendications 1 à 10, **caractérisé en ce que** le composé polyamine est choisi parmi les polymères à base d'amine, notamment ayant un poids moléculaire moyen en poids allant de 500 à 1 000 000, de préférence allant de 500 à 500 000, et préférentiellement allant de 500 à 100 000.

14. Procédé selon la revendication précédente, **caractérisé en ce que** le composé polyamine est choisi parmi les poly(alkylène (C₂-C₅) imines), et en particulier les polyéthylèneimines et les polypropylèneimines, notamment les poly(éthylène imines); la poly(allylamine) ; les polyvinylamines et leurs copolymères notamment avec des vinylamides; les copolymères vinylamine/vinylformamide ; les poly(acides aminés) porteurs des groupes NH2 comme la polylysine ; l'amino dextrane ; l'amino alcool polyvinylique, les copolymères à base d'acrylamidopropylamine; les chitosanes ;
les polydiméthylsiloxanes comprenant des groupés amine primaire en bout de chaîne ou sur des chaînes latérales, par exemple des groupes latéraux ou terminaux aminopropyle, comme par exemple ceux de formule (A) ou (B) ou (C) : 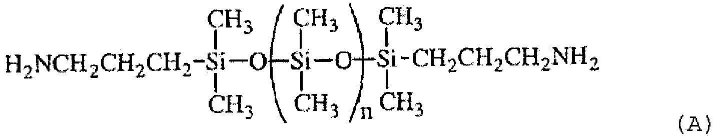 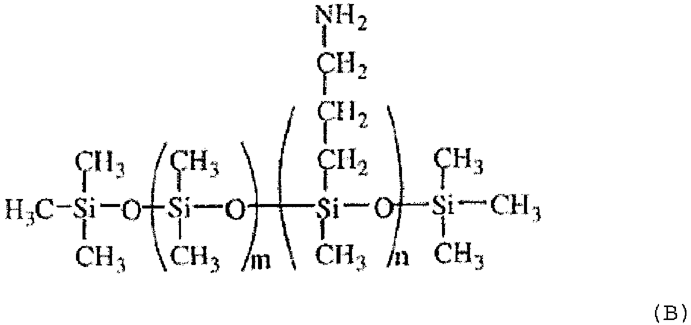
H₂NCH₂CH₂CH₂-Si(CH₃)₂-O-[Si(CH₃)₂-O]ₙ-Si(CH₃)₂C₄H₉ (C)
avec :
dans la formule (A) : la valeur de n est telle que le poids moléculaire moyen en poids de la silicone est compris entre 500 et 55 000 ;
dans la formule (B), les valeurs de n et m sont telles que le poids moléculaire moyen en poids de la silicone est compris entre 1000 et 55 000 ;
dans la formule (C), la valeur de n est telle que le poids moléculaire moyen en poids de la silicone est compris entre 500 et 3000 ;
les amodiméthicones de formule (D) : 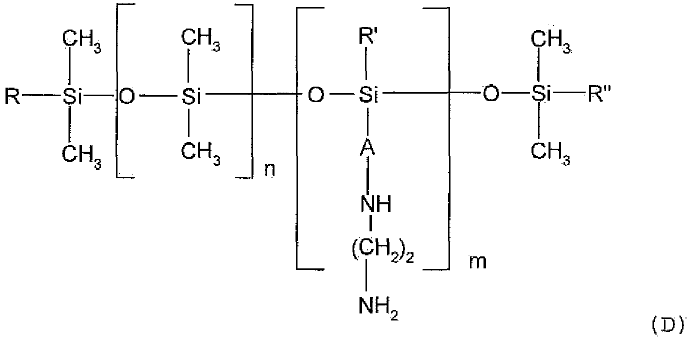 dans laquelle R, R' et R", qui peuvent être identiques ou différents, représentent chacun un groupe alkyle en C₁-C₄. ou hydroxyle, A représente un groupe alkylène en C₃ et m et n sont tels que la masse moléculaire moyenne en poids du composé est comprise entre 5 000 et 500 000 environ ;
les amodiméthicones de formule (K) : 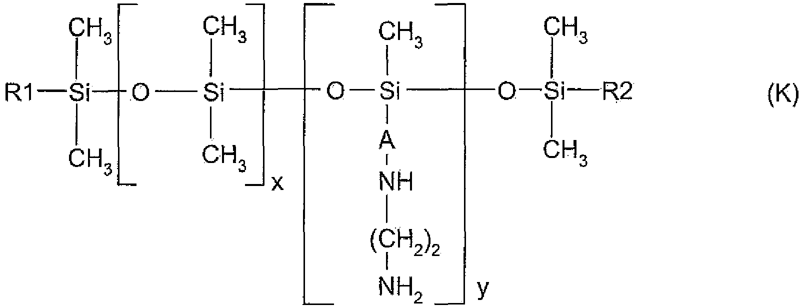 dans laquelle :
- R1 et R2, qui peuvent être identiques ou différents, de préférence identiques, représentent un groupe alkyle linéaire ou ramifié, saturé ou insaturé, comprenant de 6 à 30 atomes de carbone, de préférence de 8 à 24 atomes de carbone et préférentiellement de 12 à 20 atomes de carbone,
- A représente un groupe alkylène linéaire ou ramifié radical contenant de 2 à 8 atomes de carbone,
- x et y sont des nombres entiers allant de 1 à 5 000 ; de préférence x va de 10 à 2 000 et notamment de 100 à 1 000 ; de préférence, y va de 1 à 100 ;
préférentiellement, pour l'amodiméthicone de formule (K) :
- x va de 10 à 2 000, et notamment de 100 à 1000 ;
- y va de 1 à 100 ;
- A comprend de 3 à 6 atomes de carbone, et en particulier 4 atomes de carbone ; de préférence, A est ramifié ; préférentiellement A est choisi parmi les radicaux divalents : -CH₂CH₂CH₂- et -CH₂CH(CH₃)CH₂- ; et
- R1 et R2, qui peuvent être identiques ou différents, représentent un radical linéaire saturé, comprenant de 6 à 30 atomes de carbone, de préférence de 8 à 24 atomes de carbone, et notamment de 12 à 20 atomes de carbone, comme par exemple un groupe dodécyle, tétradécyle, pentadécyle, hexadécyle, heptadécyle, octadécyle, nonadécyle ou éicosyle ; avantageusement, R1 et R2 représentent un mélange de radicaux hexadécyle (cétyle) et octadécyle (stéaryle) (mélange appelé également cétéaryle) ;
de préférence, l'amodiméthicone de formule (K) est la bis-cétéaryle amodiméthicone ;
les polyéthers diamines et notamment les polyéthylèneglycols et/ou polypropylèneglycols α,ω-diamines ; les polytétrahydrofurannes (ou polytétraméthylèneglycols) α-ω-diamines et les polybutadiènes α,ω-diamines
les dendrimères polyamidoamine porteurs de fonctions amines terminales,
les poly(méth)acrylates ou poly (méth) acrylamides porteurs de fonctions amines primaires ou secondaires latérales, tels que le poly(3-aminopropyl)méthacrylamide et le poly(2-aminoéthyl)méthacrylate ;
de préférence les polydiméthylsiloxanes comprenant des groupes amine primaire en bout de chaîne ou sur des chaînes latérales ; les amodiméthicones de formule (K) ; les polyéthylèneglycols et/ou polypropylèneglycols α, ω-diamines ; l'éthylène diamine, la 1,3-propylènediamine, la 1,4-butylènediamine ;
préférentiellement les polydiméthylsiloxanes comprenant en bout de chaîne des groupes terminaux aminopropyle ; la bis-cétéaryle amodiméthicone, les copolymères polyéthylèneglycol/polypropylèneglycol α,ω-diamine comprenant de 2 à 50 motifs issus d'oxyde d'éthylène et de 1 à 10 motifs issus d'oxyde de propylène.

15. Procédé selon l'une quelconque des revendications précédentes, **caractérisé en ce que** le composé polyamine est utilisé selon un ratio molaire du groupe aminé du composé polyamine/groupe anhydride maléique du polymère acrylique allant de 0,01 à 10, de préférence allant de 0,1 à 5, préférentiellement allant de 0,1 à 2, et plus préférentiellement allant de 0,1 à 1.

16. Procédé selon l'une quelconque des revendications précédentes, **caractérisé en ce que** le polymère acrylique est présent en une teneur allant de 0,1 à 40 % en poids, du poids total de la composition, de préférence de 0,5 à 35 % en poids de matière active, et préférentiellement allant de 1 à 30 % en poids, et plus préférentiellement allant de 10 à 30 % en poids.

17. Procédé selon l'une quelconque des revendications précédentes, **caractérisé en ce que** la composition comprend une huile hydrocarbonée, de préférence une huile hydrocarbonée apolaire ayant de 8 à 14 atomes de carbone, préférentiellement l'isododécane.

18. Procédé selon l'une quelconque des revendications 1 à 17, **caractérisé en ce que** l'on applique d'abord sur les matières kératiniques la composition comprenant le polymère acrylique d'anhydride maléique puis on applique le composé polyamine ou une composition le contenant et comprenant un milieu physiologiquement acceptable.

19. Procédé selon l'une quelconque des revendications 1 à 17, **caractérisé en ce que** l'on applique d'abord sur les matières kératiniques le composé aminé, ou une composition le contenant et comprenant un milieu physiologiquement acceptable, puis on applique la composition comprenant le polymère acrylique d'anhydride maléique.

20. Procédé selon l'une quelconque des revendications précédentes, **caractérisé en ce qu'**il est effectué sur la peau, les lèvres, les cils, les cheveux ou les ongles.

21. Kit comprenant une première composition comprenant un polymère acrylique d'anhydride maléique tel que défini dans l'une quelconque des revendications 1 à 10 et comprenant un milieu physiologiquement acceptable, et une deuxième composition comprenant un composé polyamine tel que défini dans l'une des revendications 1 et 11 à 14 et comprenant un milieu physiologiquement acceptable, les première et deuxième compositions étant conditionnées chacune dans un ensemble de conditionnement distinct.

22. Polymère susceptible d'être obtenu par réaction d'un polymère acrylique d'anhydride maléique tel que défini dans l'une quelconque des revendications 1 à 10 avec un composé polyamine tel que défini dans l'une des revendications 1 et 11 à 14.
